# EUROPEAN PATENT SPECIFICATION

(11) **EP 2 883 569 B1**
(45) Date of publication and mention of the grant of the patent: **20.12.2017**
(21) Application number: 13756518.0
(22) Date of filing: 19.07.2013
(51) Int. Cl.: A61K 35/19, A61K 35/16, A61K 9/72

(54) **COMPOSITIONS COMPRISING PLASMA GROWTH FACTORS FOR USE IN THE TREATMENT OF NEURODEGENERATIVE DISORDERS**
ZUSAMMENSETZUNGEN PLASMA WACHSTUMSFAKTOREN ENTHALTEND ZUR VERWENDUNG BEI DER BEHANDLUNG VON NEURODEGENERATIVEN ERKRANKUNGEN
COMPOSITIONS COMPRENANT DES FACTEURS DE CROISSANCE PLASMATIQUE POUR L'UTILISATION DANS LA TRAITEMENT DES TROUBLES NEURODÉGÉNÉRATIFS

(30) Priority: 09.08.2012 ES 201200810
(43) Date of publication of application: 17.06.2015
(73) Proprietor: Biotechnology Institute, I MAS D, S.L., 01005 Vitoria (ES)
(72) Inventor: ANITUA ALDECOA, Eduardo, E-01005 - Vitoria (ES)
(74) Representative: Cueto, Sénida
(86) International application number: PCT/ES2013/000176
(87) International publication number: WO 2014/023860

(56) References cited:
- EP-A1- 1 066 838
- WO-A2-2009/016451
- WO-A2-2010/130851
- SALVADOR HARGUINDEY ET AL: "Growth and trophic factors, pH and the Na+/H+ exchanger in Alzheimer's disease, other neurodegenerative diseases and cancer: new therapeutic possibilities and potential dangers.", CURRENT ALZHEIMER RESEARCH, vol. 4, 2007, pages 53-65, XP002715417,
- EDUARDO ANITUA ET AL: "Plasma rich in growth factors: The pioneering autologous technology for tissue regeneration", JOURNAL OF BIOMEDICAL MATERIALS RESEARCH PART A, vol. 97A, no. 4, 15 June 2011 (2011-06-15) , pages 536-536, XP055084106, ISSN: 1549-3296, DOI: 10.1002/jbm.a.33053
- SALVADOR HARGUINDEY ET AL: "An integral approach to the etiopathogenesis of human neurodegenerative diseases (HNDDs) and cancer. Possible therapeutic consequences within the frame of the trophic factor withdrawal syndrome (TFWS)", NEUROPSYCHIATRIC DISEASE AND TREATMENT, vol. 4, no. 6, 1 December 2008 (2008-12-01), pages 1073-1084, XP055084108, ISSN: 1176-6328, DOI: 10.2147/NDT.S3800
- EDUARDO ANITUA ET AL: "Perspectives and challenges in regenerative medicine using plasma rich in growth factors", JOURNAL OF CONTROLLED RELEASE, ELSEVIER, AMSTERDAM, NL, vol. 157, no. 1, 6 June 2011 (2011-06-06), pages 29-38, XP028443366, ISSN: 0168-3659, DOI: 10.1016/J.JCONREL.2011.07.004 [retrieved on 2011-07-08]
- EDUARDO ANITUA ET AL: "Intranasal Delivery of Plasma and Platelet Growth Factors Using PRGF-Endoret System Enhances Neurogenesis in a Mouse Model of Alzheimer's Disease", PLOS ONE, vol. 8, no. 9, 1 September 2013 (2013-09-01), page e73118, XP055084112, ISSN: 1932-6203, DOI: 10.1371/journal.pone.0073118

## Description

### Technical field

The invention is defined in claims 1-4 and relates to a composition obtained from at least one blood compound with growth factors, to be used in treating neurodegenerative diseases via the intranasal route.

### Prior art

The preparation of compositions from growth-factor-containing blood compounds, obtained from the blood of a patient, is very widely known in prior art. Said compositions have been shown to offer very important biological properties, having to do especially with the triggering and encouraging of tissue regeneration, the reduction of pain in certain types of ailments and diseases, and many other uses. For example, among the many extensive uses of compositions obtained from blood growth-factor-rich compounds are the regeneration of alveolar bone in oral cavities in implantology and other procedures, and the regeneration of tissue in the locomotor system (muscles, joints, tendons, etc).

As for the type of known growth-factor-rich compositions, by way of example, patent application WO0044314A1 and patent application WO2010130851A2 are known, both of which belonging to the applicant of this invention. These patents refer to procedures for the preparation of an autologous blood plasma gel, rich in growth factors, from the patient's own blood. Both procedures share some common steps, such as centrifuging of the patient's blood, separating platelet-rich plasma and adding calcium chloride to the platelet-rich plasma to cause the activation of the plasma (the release of growth factors by the platelets contained in the plasma) and to cause the plasma to coagulate until it acquires a consistency similar to a gel. In another example, patent ES2221770B2 describes a procedure for the preparation of another composition obtained from a growth-factor-rich blood compound, said composition having highly beneficial biological properties and, in this case, a liquid form. Specifically, the composition is a supernatant of a growth-factor-rich blood plasma, obtained from the supernatant liquid phase that appears after causing the coagulation and subsequent retraction of said growth-factor-rich plasma. This patent also describes various uses of the supernatant, such as its use (prompted by its liquid consistency) as eye drops for the treatment of eye diseases and ailments.

It is an aim of this invention to broaden the therapeutic boundaries of the possible applications of growth-factor-rich plasma gel, of growth-factor-rich plasma supernatant obtained as defined in claims 1 and 3.

### Brief description of the invention

It is an object of the invention to provide the treatment of neurodegenerative diseases by means of a composition obtained by centrifuging blood, separating platelet-rich plasma and adding calcium chloride to the platelet-rich plasma thereby causing a release of growth factors by platelets contained in the plasma and the plasma to coagulate until it acquires a consistency similar to a gel, or by centrifuging blood, separating platelet-rich plasma, causing a coagulation and subsequent retraction of said platelet-rich-plasma and obtaining a supernatant from said retraction. The composition is configured for its use as a therapeutic substance to be administered intranasally in the treatment of a neurodegenerative disease. The means of administering the composition or the therapeutic substance, according to the invention, in order for it to reach the central nervous system in an effective manner in terms of the treatment and also in a safe manner for the patient, is the intranasal route. The intranasal route is a non-invasive access route to the central nervous system that, in contrast to invasive routes, does not require continual injections or other means of administration that cause trauma to the patient or cause secondary effects to the patient; therefore, the intranasal route allows the composition or therapeutic substance to be administered with great ease and, as a result, the treatment to be performed without difficulty. It should also be noted that the intranasal route is a very relevant non-invasive route from the perspective of having to access the central nervous system, as said access in not possible through other non-invasive routes due to the existence of a number of physiological barriers, among them the blood-brain barrier.

### Brief description of the drawings

Details of the invention can be seen in the accompanying figures, which do not intend to limit the scope of the invention:
- Figure 1 shows the results of studies measuring the formation of beta-amyloid plaques after three and six months, in APP/PS1 transgenic mice treated by means of a growth-factor-rich plasma administered by the intranasal route.
- Figure 2 shows the results of studies measuring the amount of beta-amyloid in AB40 and AB42 form after three and six months, in APP/PS1 transgenic mice treated by means of a growth-factor-rich plasma administered by the intranasal route.
- Figure 3 shows the results of studies measuring the astrogliosis after six months, in APP/PS1 transgenic mice treated by means of a growth-factor-rich plasma administered by the intranasal route.
- Figure 4 shows the results of studies measuring the neurogenesis after six months, in APP/PS1 transgenic mice treated by means of a growth-factor-rich plasma administered by the intranasal route.
- Figure 5 shows the results of two behavioural tests of APP/PS1 transgenic mice treated by means of a growth-factor-rich plasma administered by the intranasal route.

### Detailed description of the invention

There is one part of the anatomy in which it is more than likely that beneficial therapeutic effects deriving from the use of compositions obtained from blood compounds with growth factors may be detected, said part being one of the most important of all the systems to be found in the body: the central nervous system, in charge of receiving and processing the sensations registered by the senses and of accurately transmitting response orders to the various effectors. Specifically, the cells that form the central nervous system are arranged in such a way that they give rise to two highly characteristic formations: grey matter, consisting of neuronal bodies, and white matter, formed mainly by nervous prolongations (dendrites and axons), whose function is to carry the information.

Some of the most common diseases found nowadays occur in the central nervous system and especially in elderly people. Among these common diseases are neurodegenerative diseases. A neurodegenerative disease is a type of disease involving cognitive disorders, such as Alzheimer's disease, Parkinson's disease, Huntington's disease, Creutzfeldt-Jakob disease and multiple sclerosis, to name but a few. These cognitive disorders are caused by an increase in cell death processes that results in a great reduction of neuron number, behavioural changes and a general gradual degeneration that leads to the patient's death. In addition, in many of these diseases changes in the blood flow in the brain take place which cause lesions of varying degrees of importance, depending on the location and the size of the affected area and the period of time for which the patient has gone without correct treatment.

At this moment in time, a promising treatment for some of these diseases is perceived to be the administration of trophic agents (substances that impact on axonic growth and on the formation of synapses, and which enhance the survival of the cells of the central nervous system), antiapoptotic agents (substances that reduce cell death) and/or neovascularisation inducers (substances that encourage the formation of blood vessels). These substances cannot cure these diseases, but they do serve as therapeutic tools that delay the development of the diseases and thus the many cognitive changes they bring about.

However, the administration of substances to the central nervous system brings with it significant galenic and technological problems due to the anatomical features of this area and the difficulty in delivering substances to it. It should be borne in mind that the central nervous system, which consists of the brain and the spinal cord, is extremely well protected from the rest of the body and the exterior by three membranes: the dura mater (external membrane), the arachnoid mater (intermediate membrane) and the pia mater (internal membrane), collectively known as the meninges. The brain and the spinal cord are also encased in bones, namely the cranium and the spinal column respectively. The cavities of these organs (ventricles in the case of the brain and the ependimary canal in the case of the spinal cord) are filled with a colourless and transparent fluid called cephalorachidian fluid. Cephalorachidian fluid performs a wide variety of functions: it acts as a means for exchanging certain substances, as a system for eliminating residual products and for maintaining the correct ionic equilibrium and as a mechanical cushioning system.

The invention proposes, seeking to respond to the ongoing need to viably treat diseases of the central nervous system, the treatment of said diseases (or even other applicable diseases) by means of a composition obtained by centrifuging blood, separating platelet-rich plasma and adding calcium chloride to the platelet-rich plasma thereby causing a release of growth factors by platelets contained in the plasma and the plasma to coagulate until it acquires a consistency similar to a gel, or by centrifuging blood, separating platelet-rich plasma, causing a coagulation and subsequent retraction of said platelet-rich-plasma and obtaining a supernatant from said retraction. The composition is configured for its use as a therapeutic substance to be administered intranasally in the treatment of a neurodegenerative disease. The means of administering the composition, according to the invention, in order for it to reach the central nervous system in an effective manner in terms of the treatment and also in a safe manner for the patient, is administration by the intranasal route. The intranasal route is a non-invasive route providing access to the central nervous system and allows the composition or therapeutic substance to be applied with great ease and the treatment to be performed without difficulty, because, in contrast to invasive routes, it does not require continual injections or other means of administration that cause trauma to the patient, and does not cause secondary effects to the patient. It should also be noted that the intranasal route is a very important non-invasive route in terms of accessing the central nervous system, as said access in not possible through other non-invasive routes due to the existence of a number of physiological barriers. Intranasal administration is also simple, safe and easy to use, and allows the patient to self-administer the medicine without the need for other people's assistance.

The administration of the growth-factor-containing blood composition by intranasal route allows the blood composition and its therapeutic agents to reach the central nervous system and achieve its biological effects in the manner explained hereafter. The olfactory and trigeminal nerves, which innervate the nasal cavity, provide a direct connection from the nasal cavity to the central nervous system. The trigeminal nerve does not provide branches to the olfactory region but rather innervates the respiratory epithelium and the vestibule of the nasal cavity. It also establishes a connection with the central nervous system, in particular with the caudal brain region, the brain stem and the spinal cord, and transmits sensory information from the nasal cavity to these areas. These conditioning factors thus provide nasal mucus with highly unique anatomical and physiological attributes, which facilitate the introduction into the body of therapeutic means capable of reaching the central nervous system following nasal instillation and thereby avoiding the blood-brain barrier.

Intracellular, extracellular and perivascular transport mechanisms may occur in the olfactory region. The intracellular path taken by a substance towards the central nervous system is the result of said substance being collected by the dendrites of the olfactory neurons, of pinocytosis or of simple diffusion, and its subsequent transfer by the olfactory nerve. The extracellular or paracellular transport mechanism allows the substance to pass between the cells in the nasal epithelium and to rapidly enter the perineural channels (created by the surrounding olfactory cells that envelop the branches of the olfactory nerve), allowing the substance to reach the cephalorachidian fluid and the olfactory bulb. From the cephalorachidian fluid, the substance can mix with the interstitial fluid in the brain, allowing its distribution throughout the organ; in addition, the substance may enter the perivascular spaces in the nasal mucus and the brain tissue for its rapid distribution throughout the central nervous system.

The composition may be autologous (the donor and recipient are the same person) or heterologous (the donor and recipient are different people) prepared according to any applicable preparation method. The growth factors may be in released state or not. The supernatant or gel may be of recent preparation or may have been prepared beforehand and stored (for example, by means of the technique involving heat treatment, lyophilization and subsequent re-suspension proposed by patent application no. ES2369945A1, also belonging to the applicant).

In-depth study has been carried out over the last 20 years with a view to determining the substances (proteins, peptides, growth factors, etc) found in blood compositions. It is now known that plasma and, in particular, platelets contain a significant number of substances, including neurotrophic agents such as nerve growth factor (NGF), angiogenic substances or substances encouraging the formation of blood vessels such as VEGF, angiopoietins, PDGF, EGF, IGF-I, etc. and antiapoptotic agents such as VEGF.

The composition may optionally contain haematic cellular components (platelets, erythrocytes and white blood cells).

The 'therapeutic substance' is understood to be the composition itself or a substance obtained from said composition for therapeutic purposes.

The liquid nature of the supernatant, its fluidity and osmotic properties make it an ideal preparation for its administration by the nasal route.

In turn, the use of a gel-type composition provides a growth-factor transfer that lasts several weeks, which reduces the number of times that the patient has to self-administer the composition.

Experimental results are described hereafter, which show the effectiveness of neurodegenerative disease treatment by administration via the intranasal route of a composition as defined in claims 1 or 3.

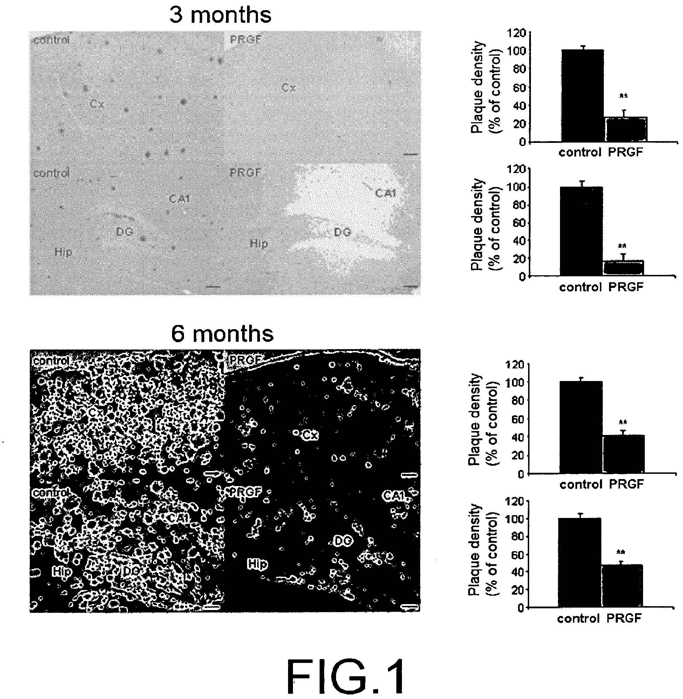

Figure 1 shows the results of studies in which the formation of beta-amyloid plaques was measured at three and six months in APP/PS1 transgenic mice; specifically, the plaque density was measured, both in animals treated by the intranasal route with a plasma rich in growth factors (PRGF) and in a control group comprised of sick, untreated animals. The PRGF used in these experiments was obtained by means of the preparation method described in US6569204. Beta-amyloid (Aβ) is a peptide of 36 to 43 amino acids that is synthesised from the amyloid precursor protein. Although it is generally known for its relationship with Alzheimer's disease, it is still not known whether it is a cause or an effect of the disease. Beta-amyloid is the main component of senile plaques (deposits found in the brain of patients with Alzheimer's disease). An increase in the overall levels of Aβ or an increase in the concentration of both Aβ40 and Aβ42 is related entirely with the pathogen of Alzheimer's disease. The data in Figure 1 corresponds both to the region of the cortex (Cx) and to the hippocampus (Hip), with the area CA1 of the hippocampus and the dentate gyrus (DG) also being shown. The results clearly show how animals receiving PRGF by the intranasal route present significantly lower levels of plaques, regardless of the location and monitoring time.

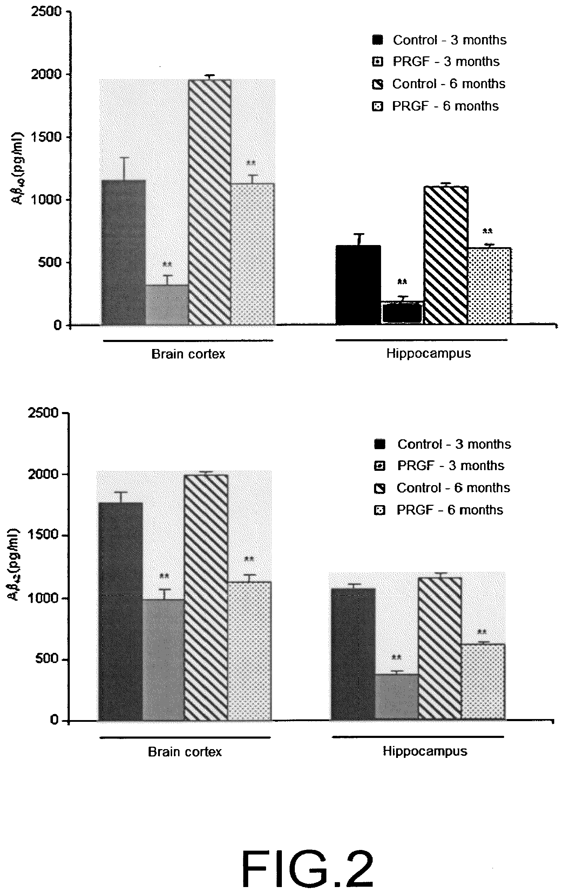

Figure 2 shows the results of studies in which the amount of beta-amyloid in Aβ40 and Aβ42 form was measured at three and six months in APP/PS1 transgenic mice treated with PRGF, in comparison with a control group comprised of sick untreated animals. The data corresponds both to the region of the cortex (Cx) and the hippocampus (Hip). The results clearly show how animals receiving PRGF by the intranasal route present significantly lower levels of both types of beta-amyloid, partly responsible for the neuronal damage, regardless of the location and monitoring time.

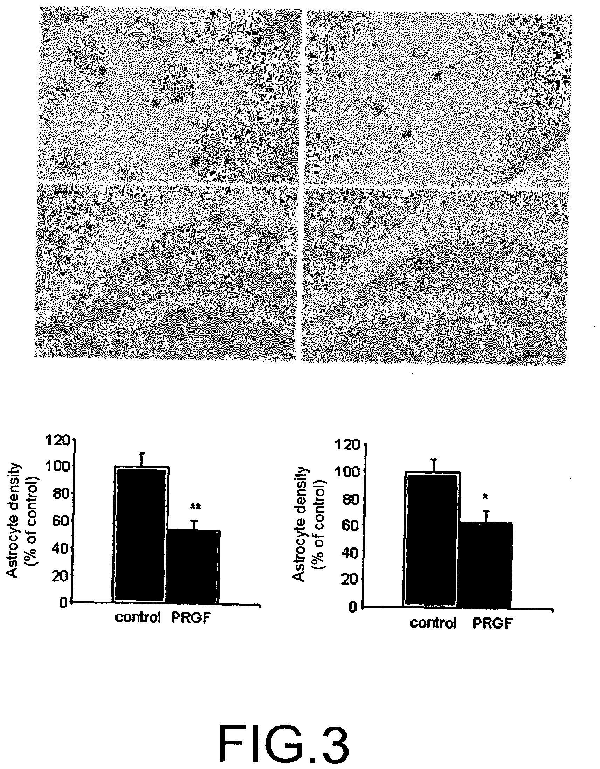

Figure 3 shows the results of studies in which the degree of astrogliosis or abnormal growth of astrocytes due to the destruction of neighbouring neurons in APP/PS1 transgenic mice treated with PRGF was measured, in comparison with a control group comprised of sick untreated animals. Astrocytes are the principal and most numerous glial cells (cells in the nervous system that act as a support for neurons and play an active part in the cerebral processing of information in the body). The presence of astrogliosis is an indicator of toxicity in the brain. In general terms, the presence of beta-amyloid plaques increases the relative level of astrocytes or astrogliosis. The data corresponds to the region of the cortex (Cx), the hippocampus (hip) and the dentate gyrus (DG). The results clearly show how animals receiving PRGF by the intranasal route present significantly lower levels of reactive astrocytes regardless of the location and monitoring time.

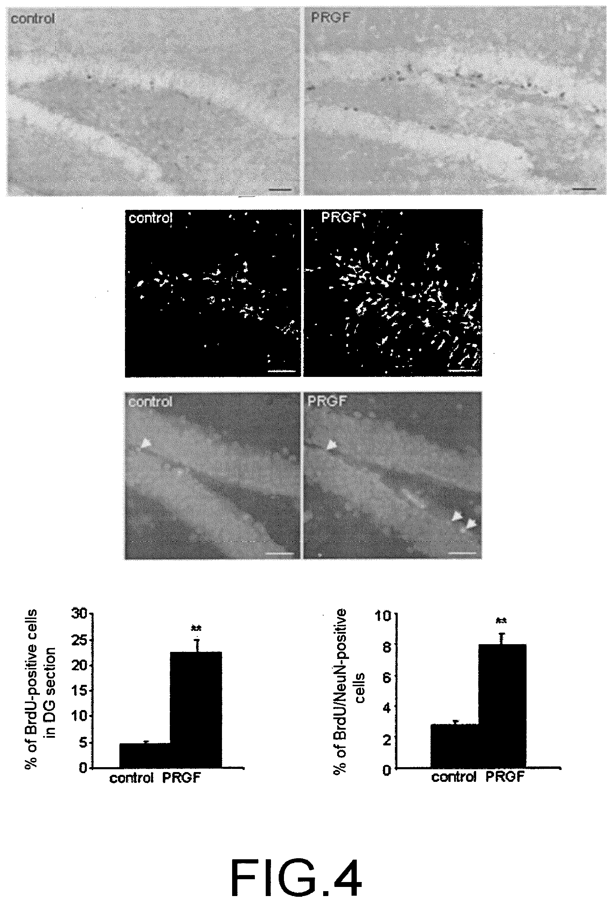

Figure 4 shows the results of studies in which neurogenesis (the differentiation of new neurons from precursor cells) was measured at six months in APP/PS1 transgenic mice treated with PRGF, in comparison with a control group comprised of sick untreated animals. The data corresponds to the region of the hippocampus (Hip). The results clearly show how animals receiving PRGF by the intranasal route present significantly more neurogenesis. Using different types of staining (corresponding to the first, second and third pair of comparative photographs) the presence of a greater number of new cells in the histologies belonging to the group treated with PRGF may be seen. Specifically, for the purpose of accurately determining the cellular phenotype of these new cells it was decided to carry out a double immunohistochemical staining (BrdU/NeuN) that allowed not only to stain the divided cells (i.e. the newly created cells) but also to stain whether they specifically are neurons or not, thanks to NeuN staining.

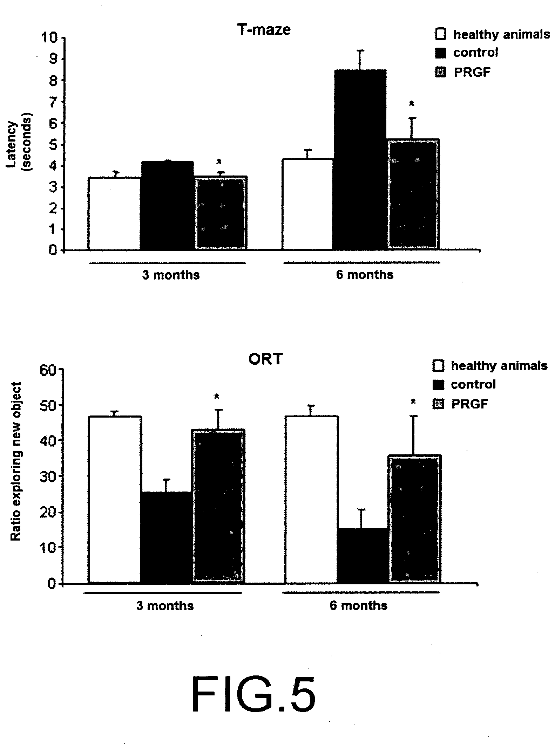

Figure 5 shows the results of two behavioural tests assessing short- and medium-term memory and other symptoms associated with Alzheimer's disease, such as apathy. Tests were performed on three groups of APP/PS1 transgenic mice: one group of healthy animals, a control group comprised of sick untreated animals (with Alzheimer's disease) and a group of animals with the same illness but treated with PRGF by the intranasal route. The data shows that the APP/PS1 transgenic mice receiving PRGF by the intranasal route show recovery values in the behavioural tests, reflecting a significant improvement in comparison to the sick untreated mice.

Specifically, in the first test the animals were placed in a T-maze in which one of the side arms of the maze was initially closed and the animals were left to complete the other arm. The animals were then placed in the maze again and the time they needed to reach the arm they were acquainted with in the previous experiment was analysed. The animals in the control group did not remember very well what they had done and demonstrated both apathy and fear, which meant that they took more time to complete the experiment. In contrast, the sick animals receiving an intranasal treatment completed the experiment in a significantly shorter time than the animals of the control group (of sick untreated animals).

Additionally, the second test or Object Recognition Test (ORT) was based on the exploration by the animal of a clearly identified central area inside a square-shaped space. Typically, a sick animal has difficulties in exploring new areas, especially if said areas are not close to the walls because of the animal being scared of the unknown. During the test, the time the animals spent in the delimited central area was recorded and compared with the total recognition time (with the graph showing the ratio of the first time and the second time, a higher ratio indicating a higher cognitive function). The behaviour of the same three groups of animals as in the previous test was measured. As can be seen, the animals treated with PRGF underwent a significant improvement in their behaviour both after three and six months in relation to the sick untreated animals (control group).

## Claims

1. Composition obtained by centrifuging blood, separating platelet-rich plasma and adding calcium chloride to the platelet-rich plasma thereby causing a release of growth factors by platelets contained in the plasma and the plasma to coagulate until it acquires a consistency similar to a gel, for its use as a therapeutic substance to be administered intranasally in the treatment of a neurodegenerative disease.

2. Composition for use according to claim 1, wherein the composition is autologous to a recipient to which the composition is to be administered intranasally.

3. Composition obtained by centrifuging blood, separating platelet-rich plasma, causing a coagulation and subsequent retraction of said platelet-rich-plasma and obtaining a supernatant from said retraction, for its use as a therapeutic substance to be administered intranasally in the treatment of a neurodegenerative disease.

4. Composition for use. according to claim 3, wherein the composition is autologous to a recipient to which the composition is to be administered intranasally.

## Patentansprüche

1. Zusammensetzung, erzielt durch das Zentrifugieren von Blut, das Abtrennen von blutplättchenreichem Plasma und das Zusetzen von Calciumchlorid zum blutplättchenreichem Plasma, womit eine Freisetzung von Wachstumsfaktoren durch im Plasma enthaltene Blutplättchen und die Gerinnung des Plasmas erreicht wird, bis es eine ähnliche Konsistenz wie ein Gel annimmt, zu deren Verwendung als therapeutische Substanz, die intranasal bei der Behandlung einer neurodegenerativen Krankheit verabreicht werden soll.

2. Zusammensetzung zur Verwendung gemäß Anspruch 1, wobei die Zusammensetzung autolog für einen Empfänger ist, an den die Zusammensetzung intranasal verabreicht werden soll.

3. Zusammensetzung, erzielt durch das Zentrifugieren von Blut, das Abtrennen von blutplättchenreichem Plasma, das Erzeugen einer Gerinnung und anschließenden Retraktion des besagten blutplättchenreichen Plasmas und die Erlangung eines Überstands aus besagter Retraktion, zu deren Verwendung als therapeutische Substanz, die intranasal bei der Behandlung einer neurodegenerativen Krankheit verabreicht werden soll.

4. Zusammensetzung zur Verwendung gemäß Anspruch 3, wobei die Zusammensetzung autolog für einen Empfänger ist, an den die Zusammensetzung intranasal verabreicht werden soll.

## Revendications

1. Composition obtenue en centrifugeant le sang, en séparant le plasma riche en plaquettes et en ajoutant du chlorure de calcium au plasma riche en plaquettes et provoquant ainsi l'émergence de facteurs de croissance par des plaquettes contenues dans le plasma et la coagulation du plasma jusqu'à ce qu'il acquière une consistance similaire à un gel, à utiliser comme formule thérapeutique à administrer par voie intranasale dans le traitement d'une maladie neurodégénérative.

2. Composition à utiliser conformément à la revendication 1, où la composition est autologue pour un destinataire auquel la composition doit être administrée par voie intranasale.

3. Composition obtenue en centrifugeant du sang, en séparant le plasma riche en plaquettes, en provoquant une coagulation et la rétraction ultérieure dudit plasma riche en plaquette et en obtenant un surnageant de ladite rétraction, à utiliser comme substance thérapeutique administrée par voie intranasale dans le traitement d'une maladie neurodégénérative.

4. Composition à utiliser conformément à la revendication 3, où la composition est autologue pour un destinataire auquel la composition doit être administrée par voie intranasale.
